# EUROPÄISCHE PATENTANMELDUNG

(11) **EP 3 023 895 A1**
(43) Veröffentlichungstag der Anmeldung: **25.05.2016**
(21) Anmeldenummer: 14194514.7
(22) Anmeldetag: 24.11.2014
(51) Int. Cl.: G06F 19/00

(54) **Verfahren zum Vergleichen von medizinischen Datensätzen**

(71) Anmelder: Siemens Aktiengesellschaft, 80333 München (DE)
(72) Erfinder: Hewett, Andrew John, 91058 Erlangen (DE); Seifert, Sascha, 75203 Königsbach-Stein (DE)

(57) **Zusammenfassung**

Die vorliegende Erfindung betrifft Verfahren zum Vergleichen von medizinischen Daten, mit den Schritten eines Übermittelns (S101) von ersten medizinischen Datensätzen aus einem ersten Netzwerk an einen zentralen Datenspeicher; eines Übermittelns (S102) von zweiten medizinischen Datensätzen aus einem zweiten Netzwerk an den zentralen Datenspeicher; eines Extrahierens (S103) von ersten Schlüsseldaten aus den ersten medizinischen Datensätzen in dem zentralen Datenspeicher; eines Extrahierens (S104) von zweiten Schlüsseldaten aus den zweiten medizinischen Datensätzen in dem zentralen Datenspeicher; und eines Berechnens (S105) von Vergleichswerten auf Basis der ersten Schlüsseldaten und der zweiten Schlüsseldaten.

## Beschreibung

Die vorliegende Erfindung betrifft ein Verfahren zum Vergleichen von medizinischen Daten.

Heutzutage sammeln medizinische Einrichtungen beispielsweise Daten über eine Verwendung oder Auslastung von Untersuchungsgeräten. Die gewonnenen Daten können mit Daten verglichen werden, die in der Literatur verfügbar sind. Ein direkter Vergleich von mehreren Radiologieeinrichtungen mit unterschiedlichen Profilen einer Patientenverteilung, Untersuchungsarten und Untersuchungsgeräten von unterschiedlichen Herstellern kann nicht durchgeführt werden.

Es ist die Aufgabe der vorliegenden Erfindung, die Vergleichbarkeit von medizinischen Daten auf einfache technische Weise zu erhöhen.

Diese Aufgabe wird durch einen Gegenstand nach dem unabhängigen Anspruch gelöst. Vorteilhafte Ausführungsformen sind Gegenstand der abhängigen Ansprüche, der Beschreibung und der Zeichnungen.

Gemäß einem ersten Aspekt wird die Aufgabe durch ein Verfahren zum Vergleichen von medizinischen Daten gelöst, mit den Schritten eines Übermittelns von ersten medizinischen Datensätzen aus einem ersten Netzwerk an einen zentralen Datenspeicher; eines Übermittelns von zweiten medizinischen Datensätzen aus einem zweiten Netzwerk an den zentralen Datenspeicher; eines Extrahierens von ersten Schlüsseldaten aus den ersten medizinischen Datensätzen in dem zentralen Datenspeicher; eines Extrahierens von zweiten Schlüsseldaten aus den zweiten medizinischen Datensätzen in dem zentralen Datenspeicher; und eines Berechnens von Vergleichswerten auf Basis der ersten Schlüsseldaten und der zweiten Schlüsseldaten. Durch das Verfahren werden zunächst medizinische Datensätzen als Basisdaten gesammelt, um Schlüsseldaten zu berechnen. Die Schlüsseldaten werden aggregiert und harmonisiert. Die Schlüsseldaten werden mit den Schlüsseldaten anderer medizinischer Einrichtungen durch ein automatisiertes System vergleichbar gemacht. Dadurch wird der technische Vorteil erreicht, dass sich die Datensätze mit einem geringen Einsatz an Ressourcen zentral vergleichen lassen.

Die Schlüsseldaten umfassen beispielsweise Informationen über eine Dauer der Untersuchung, eine Leerstandzeit zwischen den Untersuchungen oder eine technische Spezifikation des Untersuchungsgerätes. Die Schlüsseldaten können ebenfalls Informationen über ein Untersuchungsprotokoll, eine bildgebende Modalität, ein Modell eines Untersuchungsgerätes, ein durchgeführtes Untersuchungsverfahren oder einen untersuchten Körperteil umfassen.

In einer vorteilhaften Ausführungsform des Verfahrens werden die ersten und die zweiten Schlüsseldaten auf Basis eines Untersuchungsprotokolls, einer bildgebenden Modalität, eines Modells eines Untersuchungsgeräts, eines durchgeführten Untersuchungsverfahrens oder eines untersuchten Körperteils extrahiert.

In einer weiteren vorteilhaften Ausführungsform des Verfahrens werden die ersten Schlüsseldaten und die zweiten Schlüsseldaten statistisch gemittelt, um die Vergleichswerte zu berechnen. Dadurch wird beispielsweise der technische Vorteil erreicht, dass Vergleichswerte statistisch gestreuter Schlüsseldaten mit wenigen Rechenschritten erzeugt werden können.

In einer weiteren vorteilhaften Ausführungsform des Verfahrens werden die Schlüsseldaten der ersten medizinischen Datensätze und der zweiten medizinischen Datensätze in dem zentralen Datenspeicher gespeichert. Dadurch wird beispielsweise der technische Vorteil erreicht, dass die Schlüsseldaten dauerhaft in dem zentralen Datenspeicher verfügbar sind. In einer weiteren vorteilhaften Ausführungsform des Verfahrens werden die Vergleichswerte jeweils für das erste Netzwerk und das zweite Netzwerk berechnet. Dadurch wird beispielsweise der technische Vorteil erreicht, dass die medizinischen Einrichtungen die Netzwerke betreiben, verglichen werden können.

In einer weiteren vorteilhaften Ausführungsform des Verfahrens werden die ersten und die zweiten Datensätze vor einer Übermittlung an den zentralen Datenspeicher anonymisiert. Dadurch wird beispielsweise der technische Vorteil erreicht, dass personalisierte Datensätze zur Berechnung der Vergleichswerte herangezogen werden können.

In einer weiteren vorteilhaften Ausführungsform des Verfahrens ist der zentrale Datenspeicher eine Datenbank. Dadurch wird beispielsweise der technische Vorteil erreicht, dass ein schneller Zugriff auf die Schlüsseldaten ermöglicht wird.

In einer weiteren vorteilhaften Ausführungsform des Verfahrens werden die Vergleichswerte an das erste Netzwerk oder das zweite Netzwerk übermittelt. Dadurch wird beispielsweise der technische Vorteil erreicht, dass die medizinischen Einrichtungen die Vergleichswerte auswerten können.

In einer weiteren vorteilhaften Ausführungsform des Verfahrens werden die Vergleichswerte durch eine Anzeigeeinrichtung angezeigt. Dadurch wird beispielsweise der technische Vorteil erreicht, dass die Vergleichswerte einem Benutzer dargestellt werden können.

In einer weiteren vorteilhaften Ausführungsform des Verfahrens umfassen das erste oder das zweite Netzwerk eine bildgebende Modalität zum Erzeugen der Datensätze. Dadurch wird beispielsweise der technische Vorteil erreicht, dass die Datensätze direkt von der medizinischen Modalität an den zentralen Datenspeicher übertragen werden können.

In einer weiteren vorteilhaften Ausführungsform des Verfahrens erfolgt eine Steuerung der bildgebenden Modalität auf Basis der berechneten Vergleichswerte. Dadurch wird beispielsweise der technische Vorteil erreicht, dass eine geschlossene Schleife entsteht, durch die eine Verbesserung einer Steuerung bildgebenden Modalität erreicht wird.

In einer weiteren vorteilhaften Ausführungsform des Verfahrens umfassen das erste oder das zweite Netzwerk ein lokales Bildablage- und Kommunikationssystem. Dadurch wird beispielsweise der technische Vorteil erreicht, dass die Bilder medizinischer Datensätze lokal gespeichert und Bereitgehalten werden können.

In einer weiteren vorteilhaften Ausführungsform des Verfahrens werden die Vergleichswerte in einer Datenbank gespeichert. Dadurch wird beispielsweise der technische Vorteil erreicht, dass ein schneller Zugriff auf Vergleichswerte erfolgt.

In einer weiteren vorteilhaften Ausführungsform des Verfahrens sind die ersten und die zweiten Datensätze DICOM-Dateien. Dadurch wird beispielsweise der technische Vorteil erreicht, dass die Datensätze in einem besonders geeigneten Format gespeichert sind.

Gemäß einem zweiten Aspekt wird die Aufgabe durch ein Computersystem zum Vergleichen von medizinischen Daten gelöst, mit einem ersten Netzwerk zum Übermitteln von ersten medizinischen Datensätzen an einen zentralen Datenspeicher; einem zweiten Netzwerk zum Übermitteln von zweiten medizinischen Datensätzen an den zentralen Datenspeicher; einer Extraktionseinrichtung zum Extrahieren von ersten Schlüsseldaten aus den ersten medizinischen Datensätzen in dem zentralen Datenspeicher und zum Extrahieren von zweiten Schlüsseldaten aus den zweiten medizinischen Datensätzen in dem zentralen Datenspeicher; und einer Vergleichswerterzeugungseinrichtung zum Berechnen von Vergleichswerten auf Basis der ersten Schlüsseldaten und der zweiten Schlüsseldaten. Dadurch werden beispielsweise die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:
- Fig. 1: ein Blockdiagramm eines Verfahrens zum Vergleichen von medizinischen Daten; und
- Fig. 2: eine schematische Darstellung eines Computersystems zum Vergleichen von medizinischen Daten.

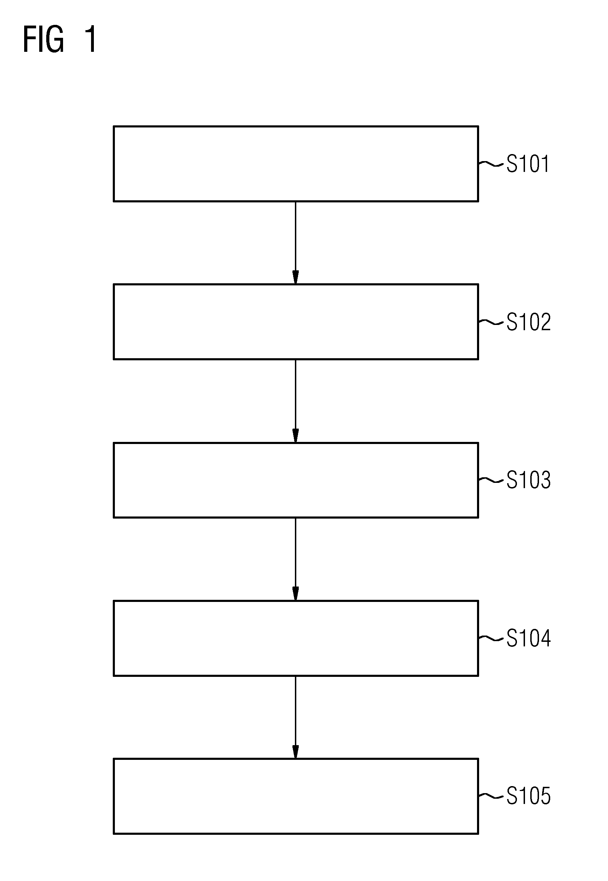

Fig. 1 zeigt ein Blockdiagramm eines Verfahrens zum Vergleichen von medizinischen Daten. Das Verfahren umfasst den Schritt S101, bei dem erste medizinische Datensätze aus einem ersten Computernetzwerk einer medizinischen Einrichtung, wie beispielsweise einem Krankenhaus, an einen zentralen Datenspeicher übermittelt werden. Der zentrale Datenspeicher ist beispielsweise durch eine Cloud im Internet oder eine Datenbank gebildet. Das Computernetzwerk ist ein Zusammenschluss verschiedener technischer, primär selbständiger elektronischer Systeme, wie beispielsweise Computer oder medizinische Geräte, das die Kommunikation der einzelnen Systeme untereinander ermöglicht.

In Schritt S102 werden zweite medizinische Datensätze aus einem zweiten Computernetzwerk 101-2 einer anderen medizinischen Einrichtung an den zentralen Datenspeicher 103 übermittelt. Der zentrale Datenspeicher 103 umfasst beispielsweise ein Computersystem mit einer Festplatte oder eine Datenbank. In Schritt S103 extrahiert der zentrale Datenspeicher erste Schlüsseldaten aus den ersten medizinischen Datensätzen. In Schritt S104 extrahiert der zentrale Datenspeicher zweite Schlüsseldaten aus den zweiten medizinischen Datensätzen. Anschließend werden durch den zentralen Datenspeicher Vergleichswerte (Benchmarks) auf Basis der ersten Schlüsseldaten und der zweiten Schlüsseldaten berechnet.

Die Vergleichswerte können beispielsweise durch eine gewichtete oder ungewichtete statistische Mittelung der Schlüsseldaten berechnet werden. Die zentral mit geringen Ressourcen berechneten Vergleichswerte erlauben anschließend einen einheitlichen Vergleich der beiden medizinischen Einrichtungen.

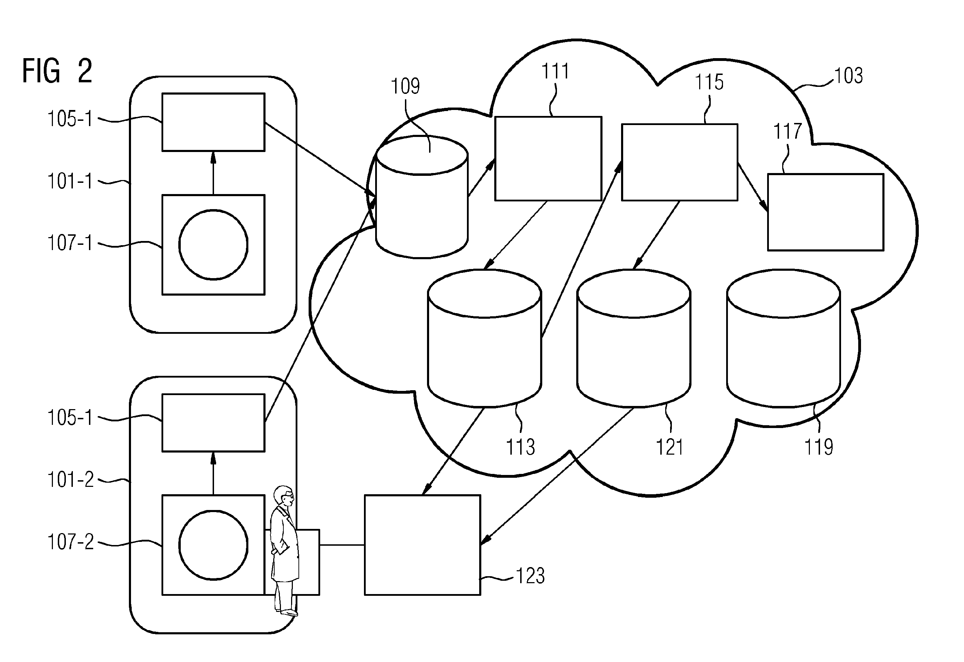

Fig. 2 zeigt eine schematische Darstellung eines Computersystems 100 zum Vergleichen von medizinischen Datensätzen. Die Datensätze werden von den lokalen Netzwerken 101-1 und 101-2 an einen zentralen Datenspeicher 103 übermittelt. Die Netzwerke 101-1 und 101-2 sind beispielsweise jeweils innerhalb eines Krankenhauses angeordnet und umfassen jeweils eine Anwendung 105-1 und 105-2 und eine bildgebende Modalität 107-1 und 107-2, wie beispielsweise einen MR-Scanner oder CT-Scanner.

In Schritt S201 sendet die medizinische bildgebende Modalität 107-1 und 107-2 kontinuierlich medizinische Datensätze, beispielsweise DICOM-Dateien, an die Anwendung 105-1 und 105-2, die innerhalb der Einrichtung lokalisiert ist. Daneben kann die Anwendung 105-1 und 105-2 die medizinischen Datensätze aus einer alternativen Quelle abrufen, wie beispielsweise aus einem lokalen Bildablage- und Kommunikationssystem (PACS - Picture Archiving and Communication System).

In Schritt S202 entfernen die Anwendungen 105-1 und 105-2 persönliche Daten (PHI - Protected Health Information) aus den medizinischen Datensätzen und senden diese an den zentralen Datenspeicher 109. In Schritt S203 werden in einer Extraktionseinrichtung 111 die heraufgeladenen Datensätze aus dem zentralen Datenspeicher 109 ausgelesen und die Schlüsseldaten (KPI - Key Performance Indicators) aus den Datensätzen extrahiert. Die Extraktionseinrichtung 111 ist beispielsweise durch eine Schaltung oder ein Programm gebildet, dass in dem Datenspeicher 103 ausgeführt wird. In Schritt S204 werden die extrahierten Schlüsseldaten in einer Datenbank 113 gespeichert.

In Schritt S205 werden die Schlüsseldaten aus der Datenbank 113 durch eine Vergleichswerterzeugungseinrichtung 115 ausgelesen. Die Vergleichswerterzeugungseinrichtung 115 ist beispielsweise durch eine Schaltung oder ein Programm gebildet, dass in dem Datenspeicher 103 ausgeführt wird. Danach werden Vergleichswerte für das erste Netzwerk 101-1 und das zweite Netzwerk 101-2 auf Basis der durchschnittlichen Schlüsseldaten berechnet. Daneben können die Vergleichswerte nach Land, geographischer Region oder nach medizinischer Einrichtung berechnet werden.

In Schritt S206 berechnet ein konfigurierbarer Vergleichswert-Analysator 117 Gruppen medizinischer Einrichtungen gemäß vorgegebenen Einstellungswerten eines Benutzers. Zu diesem Zweck verwendet der Vergleichswert-Analysator 117 eine Datenbank 119, in der alle Zugehörigkeiten der medizinischen Einrichtungen gespeichert sind.

Zusätzlich berechnet der Vergleichswert-Analysator 117 mehrere Ähnlichkeitswerte für die medizinischen Einrichtungen, die aus den Daten der medizinischen Einrichtung abgeleitet werden, wie beispielsweise für Geräte, Patienten oder Fälle. Dieser Ähnlichkeitswert dient dazu, eine optimale medizinische Partnereinrichtung zu identifizieren, die hinsichtlich der Struktur oder Leistungsfähigkeit ähnlich ist.

In Schritt S207 werden die Vergleichswerte aktualisiert, die in der Datenbank 121 gespeichert sind. In Schritt S208 und S209 visualisiert eine Anwendung 123 die Schlüsseldaten und vergleicht diese mit den zuvor berechneten Vergleichswerten. Die Vergleichsdaten, die durch die Anwendung 123 bereitgestellt werden, können dazu dienen, die Auslastung bildgebender Modalitäten 107-1 und 107-2 zu optimieren, mit denen die Datensätze erzeugt worden sind.

Die Vergleichswerte sind nahezu in Echtzeit verfügbar und Benutzer des Systems haben einen Zugang zu aktuellen Vergleichswerten. Insbesondere sind die Vergleichswerte für unterschiedliche Gruppen medizinischer Einrichtungen verfügbar.

Ein diagnostisches Bildzentrum als medizinische Einrichtung kann sich auf Basis der gemittelten Vergleichswerte mit einem anderen diagnostischen Bildzentrum in der gleichen Region, dem gleichen Land oder ähnlichen Ländern vergleichen. Ein Universitätskrankenhaus als medizinische Einrichtung kann sich auf Basis der gemittelten Vergleichswerte mit anderen Universitätskrankenhäusern weltweit vergleichen.

Medizinische Einrichtungen können die Erlaubnis von anderen befreundeten Einrichtungen einholen, um einen individuellen Vergleich durchzuführen. Medizinische Einrichtungen können Gruppen befreundeter Einrichtungen bilden, die eine Vergleichsgemeinschaft bilden.

Der Vergleichswert-Analysator 117 erlaubt es, automatisch Vergleichspartner mit gleicher Leistungsfähigkeit durch Vergleichen der Schlüsselwerte zu identifizieren, wie beispielsweise medizinische Einrichtungen oder Gruppen aus diesen. Zudem ist es möglich, Partner mit ähnlichen Verbesserungsinteressen zu finden.

Zudem kann eine ähnliche Umgebung bildgebender Modalitäten 107-1 und 107-2 identifiziert werden, wie beispielsweise gleiche Modelle und Anzahl der Scanner, ähnliche Krankheitsfälle, abgeleitete Untersuchungsprotokolle, Untersuchungsreihenfolgen, ähnliches Alter der Patienten oder Geschlechtsstruktur.

Der Vergleichswert-Analysator 117 kann erweitert werden, um Berechnungen der Schlüsselwerte umzukehren. Bei gegebenen Schlüsselwerten, beispielsweise aus den Vergleichswerten und bei bekannten medizinischem Gerät, Patient oder Fallstruktur können Maßnahmen ermittelt werden, die dazu beitragen, die Ziele zu erreichen.

Ein zusätzliches Merkmal des Vergleichswert-Analysators 117 ist es, den Benutzer zu befähigen, Änderungen von Arbeitsflüssen oder eine institutionelle Struktur zu simulieren und die Wirkung zu messen. Dies kann beispielsweise durch Vergleich mit dem ähnlichsten Partner durchgeführt werden, nachdem die Änderung durchgeführt worden ist. Die Schlüsseldaten dieses Partners können zurückgemeldet werden, die dann als Prognose-Schlüsseldaten verwendet werden.

Durch das Verfahren können medizinische Institutionen weltweit medizinische Datensätze bereitstellen, aus denen Schlüsseldaten extrahiert werden. Aus den extrahierten Schlüsseldaten werden anschließend Vergleichswerte berechnet. Die Schlüsseldaten können für ein medizinisches Personal visualisiert werden und mit den Vergleichswerten verglichen werden.

Das Verfahren stellt ein verteiltes System bereit, das mehrere lokale Zentren miteinander verbindet und die medizinischen Datensätze von Patienten, Untersuchungen und Geräten beurteilt, um Schlüsseldaten zu berechnen, diese zu integrieren und an den zentralen Datenspeicher 103 heraufzuladen. Die Schlüsseldaten können mit Schlüsseldaten anderer Einrichtungen verglichen werden und die Vergleichsergebnisse können zurückübermittelt werden. Die Schlüsseldaten bilden die Basis eine Betriebseffizienz einer medizinischen Einrichtung technisch zu verbessern, wie beispielsweise eine Radiologieabteilung eines Krankenhauses.

Zum Beispiel kann die effiziente Auslastung von medizinischer Ausrüstung, wie beispielsweise CT- oder MR-Scannern auf Basis der Schlüsseldaten beurteilt werden. Die Schlüsseldaten umfassen beispielsweise Informationen über eine Dauer der Untersuchungen, eine Leerstandzeit zwischen den Untersuchungen oder eine technische Spezifikation des Untersuchungsgerätes.

Die Schlüsseldaten können ebenfalls Informationen über ein Untersuchungsprotokoll, eine bildgebende Modalität, ein Modell eines Untersuchungsgerätes, ein durchgeführtes Untersuchungsverfahren oder einen untersuchten Körperteil umfassen. Diese Schlüsseldaten können zum Vergleich mit Vergleichswerten herangezogen werden, die aus anderen medizinischen Einrichtungen stammen.

Dadurch kann die Frage beantwortet werden, ob die medizinische Einrichtung technisch besser oder schlechter als andere ähnliche Einrichtungen arbeitet und welche technischen Verfahren effizienter gestaltet werden können. Zudem können optimale Vergleichspartner identifiziert und Interessengruppen gebildet werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

### Bezugszeichenliste

- 100: Computersystem
- 101-1 , 101-2: Netzwerk
- 103: Datenspeicher
- 105-1, 105-2: Anwendung
- 107-1, 107-2: Modalität
- 109: Datenspeicher
- 111: Extraktionseinrichtung
- 113: Datenbank
- 115: Vergleichswerterzeugungseinrichtung
- 117: Vergleichswert-Analysator
- 119: Datenbank
- 121: Datenbank
- 123: Anwendung

## Patentansprüche

1. Verfahren zum Vergleichen von medizinischen Daten, mit den Schritten:
Übermitteln (S101) von ersten medizinischen Datensätzen aus einem ersten Netzwerk (101-1) an einen zentralen Datenspeicher (103);
Übermitteln (S102) von zweiten medizinischen Datensätzen aus einem zweiten Netzwerk (101-2) an den zentralen Datenspeicher (103);
Extrahieren (S103) von ersten Schlüsseldaten aus den ersten medizinischen Datensätzen in dem zentralen Datenspeicher (103);
Extrahieren (S104) von zweiten Schlüsseldaten aus den zweiten medizinischen Datensätzen in dem zentralen Datenspeicher (103); und
Berechnen (S105) von Vergleichswerten auf Basis der ersten Schlüsseldaten und der zweiten Schlüsseldaten.

2. Verfahren nach Anspruch 1, wobei die ersten und die zweiten Schlüsseldaten auf Basis eines Untersuchungsprotokolls, einer bildgebenden Modalität, eines Models eines Untersuchungsgeräts, eines durchgeführten Untersuchungsverfahrens oder eines untersuchten Körperteils extrahiert werden.

3. Verfahren nach einem der vorangehenden Ansprüche, wobei die ersten Schlüsseldaten und die zweiten Schlüsseldaten statistisch gemittelt werden, um die Vergleichswerte zu berechnen.

4. Verfahren nach einem der vorangehenden Ansprüche, wobei die Schlüsseldaten der ersten medizinischen Datensätze und der zweiten medizinischen Datensätze in dem zentralen Datenspeicher (103) gespeichert werden.

5. Verfahren nach einem der vorangehenden Ansprüche, wobei die Vergleichswerte jeweils für das erste Netzwerk und das zweite Netzwerk berechnet werden.

6. Verfahren nach einem der vorangehenden Ansprüche, wobei die ersten und die zweiten Datensätze vor einer Übermittlung an den zentralen Datenspeicher (103) anonymisiert werden.

7. Verfahren nach einem der vorangehenden Ansprüche, wobei der zentrale Datenspeicher (103) eine Datenbank ist.

8. Verfahren nach einem der vorangehenden Ansprüche, wobei die Vergleichswerte an das erste Netzwerk (101-1) oder das zweite Netzwerk (101-2) übermittelt werden.

9. Verfahren nach einem der vorangehenden Ansprüche, wobei die Vergleichswerte durch eine Anzeige angezeigt werden.

10. Verfahren nach einem der vorangehenden Ansprüche, wobei das erste oder das zweite Netzwerk (101-1, 101-2) eine bildgebende Modalität (107-1, 107-2) zum Erzeugen der Datensätze umfassen.

11. Verfahren nach Anspruch 10, wobei eine Steuerung der bildgebenden Modalität (107-1, 107-2) auf Basis der berechneten Vergleichswerte erfolgt.

12. Verfahren nach einem der vorangehenden Ansprüche, wobei das erste oder das zweite Netzwerk (101-1, 101-2) ein lokales Bildablage- und Kommunikationssystem umfassen.

13. Verfahren nach einem der vorangehenden Ansprüche, wobei die Vergleichswerte in einer Datenbank (121) gespeichert werden.

14. Verfahren nach einem der vorangehenden Ansprüche, wobei die ersten und die zweiten Datensätze DICOM-Dateien sind.

15. Computersystem (100) zum Vergleichen von medizinischen Daten, mit:
einem ersten Netzwerk (101-1) zum Übermitteln von ersten medizinischen Datensätzen an einen zentralen Datenspeicher (103);
einem zweiten Netzwerk (101-2) zum Übermitteln (S102) von zweiten medizinischen Datensätzen an den zentralen Datenspeicher (103);
einer Extraktionseinrichtung (111) zum Extrahieren von ersten Schlüsseldaten aus den ersten medizinischen Datensätzen in dem zentralen Datenspeicher (103) und zum Extrahieren von zweiten Schlüsseldaten aus den zweiten medizinischen Datensätzen in dem zentralen Datenspeicher (103); und
einer Vergleichswerterzeugungseinrichtung (115) zum Berechnen von Vergleichswerten auf Basis der ersten Schlüsseldaten und der zweiten Schlüsseldaten.
